# EUROPEAN PATENT APPLICATION

(11) **EP 2 017 245 A1**
(43) Date of publication of application: **21.01.2009**
(21) Application number: 07743041.1
(22) Date of filing: 09.05.2007
(51) Int. Cl.: C07C 2/50, C07C 13/605, C07C 13/61

(54) **PROCESS FOR PRODUCTION OF DICYCLOPENTADIENE**

(30) Priority: 10.05.2006 JP 2006131757
(71) Applicant: Idemitsu Kosan Co., Ltd., Chiyoda-ku Tokyo 100-8321 (JP)
(72) Inventor: HOSOTANI, Noriaki, Chiba 299-0193 (JP)
(74) Representative: Vossius & Partner
(86) International application number: PCT/JP2007/059606
(87) International publication number: WO 2007/129736

(57) **Abstract**

[Problem] To provide a trimethylenenorbornane production process capable of producing trimethylenenorbornane, which serves as a raw material of adamantane, with a high efficiency in a raffinate or a heavy raffinate heavy, through high efficiency production of dicyclopentadiene, which serves as a raw material of trimethylenenorbornane, without additionally using a dicyclopentadiene hydrogenation unit or a cyclopentadiene dimerization. column.

[solution] A process for the production of dicyclopentadiene is provided which includes introducing a liquid feed containing cyclopentadiene and a C₆ to C₈ fraction into a cyclopentadiene recovery column and carrying out distillation of a fluid containing dicyclopentadiene, obtained by dimerization of at least part of the cyclopentadiene, the cyclopentadiene and the C₆ to C₈ fraction, in which an overhead liquid containing cyclopentadiene and a bottom liquid containing dicyclopentadiene are obtained from the top and the bottom of the recovery column, respectively, and the overhead liquid is refluxed so that a cyclopentadiene content of the overhead liquid is 80 to 99% by mass.

## Description

### [Technical Field]

The present invention relates to a process for the production of dicyclopentadiene.

### [Background Art]

Adamantane is a stable, highly symmetrical compound in which four cyclohexane rings are condensed to form a cage-like structure. It is known that adamantane, which has such a specific adamantane skeleton and which shows peculiar functions is useful as a lubricant or as a raw material for agricultural and medical materials and highly functional industrial materials.
As a method for producing adamantane, a process is generally adopted in which trimethylenenorbornane obtained by hydrogenating dicyclopentadiene is isomerized.
As a catalyst used in such an isomerization reaction, generally known are aluminum chloride (for example, Patent Documents 1 and 2) and a cation-exchanged zeolite on which an active metal such as platinum, rhenium, nickel or cobalt is supported by impregnation (for example, Patent Document 3).
Conventional processes in which the above-described aluminum chloride catalyst or solid catalyst is used, however, have a problem that the obtained adamantane has inevitably a high cost because trimethylenenorbornane obtained by hydrogenating expensive dicyclopentadiene is used as the starting raw material.

With a view toward solving the above problem, a process for producing adamantane is proposed which is based on the observation that trimethylenenorbornane is contained in a raffinate obtained from a platfinate and which includes isomerizing trimethylenenorbornane using the raffinate as a raw material (for example, Patent Document 4).
According to the production process, it is possible to produce high purity adamantane at a low cost with a high efficiency and to solve the aforementioned problem. The trimethylenenorbornane used as a raw material, however, has been produced as a secondary product in a plant for hydrogenation of thermally cracked gasoline. Therefore, from the standpoint of adamantane production efficiency, the trimethylenenorbornane feed cannot necessarily be said to provide the optimum composition. Trimethylenenorbornane is obtained from dicyclopentadiene as a raw material which in turn is produced by dimerization of cyclopentadiene. The thermally cracked gasoline hydrogenation plant, however, has never been operated in such a manner as to produce, with high efficiency, dicyclopentadiene, in other words to produce, with high efficiency, trimethylenenorbornane which is contained in a raffinate and which serves as a raw material of adamantane. A similar problem exists when a heavy raffinate heavy which is a fraction obtained by removing a light gas fraction from the raffinate is used as a raw material.

[Patent Document 1] Japanese Unexamined Patent Application Publication No. S50-71663
[Patent Document 2] Japanese Unexamined Patent Application Publication No. 2000-143556
[Patent Document 3] Japanese Examined Patent Publication No. S52-2909
[Patent Document 4] Pamphlet of International Publication No. WO05/058779

### [Disclosure of the Invention]

### [Problem to be Solved by the Invention]

Under the above-mentioned circumstance, the present invention has as its object the provision of a process for the production of dicyclopentadiene, which serves as a raw material of trimethylenenorbornane, with a high efficiency without additionally using a dicyclopentadiene hydrogenation unit or a cyclopentadiene dimerization column so that trimethylenenorbornane, which serves as a raw material of adamantane, can be produced in a raffinate or a heavy raffinate heavy with a high efficiency.

### [Means for Solving the Problem]

The present inventors have made an earnest study with a view toward accomplishing the above object and, as a result, have found that trimethylenenorbornane which serves as a raw material of adamantane can be produced, with a high efficiency, in a raffinate or a heavy raffinate heavy through high efficiency production of dicyclopentadiene, which serves as a raw material of trimethylenenorbornane, by operating a cyclopentadiene recovery column in specific operation conditions without additionally using a dicyclopentadiene hydrogenation unit or a cyclopentadiene dimerization column in the production process of a raffinate or a heavy raffinate heavy from a thermally cracked gasoline obtained by cracking a petroleum hydrocarbon oil. The present invention has been completed based on such a finding.
That is, the present provides the following dicyclopentadiene processes:
(1) A process for the production of dicyclopentadiene, comprising introducing a liquid feed containing Cyclopentadiene and a C₆ to C₈ fraction into a cyclopentadiene recovery column and carrying out distillation of a fluid containing dicyclopentadiene, obtained by dimerization of at least part of the cyclopentadiene, the cyclopentadiene and the C₆ to C₈ fraction, wherein an overhead liquid containing cyclopentadiene and a bottom liquid containing dicyclopentadiene are obtained from the top and the bottom of the recovery column, respectively, and the overhead liquid is refluxed so that a cyclopentadiene content of the overhead liquid is 80 to 99% by mass.
(2) The process for the production of dicyclopentadiene as recited in (1), wherein a column bottom temperature of the recovery column is 50 to 120°C.
(3) The process for the production of dicyclopentadiene as recited in (1) or (2), wherein the liquid feed containing cyclopentadiene and a C₆ to C₈ fraction has a cyclopentadiene content of 2 to 10% by mass.
(4) A process for producing trimethylenenorbornane comprising the process for the production of dicyclopentadiene as recited in any one of (1) to (3).

### [Effect of the Invention]

According to the process for the production of dicyclopentadiene of the present invention, trimethylenenorbornane which serves as a raw material of adamantane can be produced, with a high efficiency, in a raffinate or a heavy raffinate heavy through high efficiency production of dicyclopentadiene, which serves as a raw material of trimethylenenorbornane, without additionally using a dicyclopentadiene hydrogenation unit or a cyclopentadiene, dimerization column.

### [Best Mode for Carrying Out the Invention]

The process for the production of dicyclopentadiene according to the present invention comprises introducing a liquid feed containing cyclopentadiene, and a C₆ to C₈ fraction into a cyclopentadiene recovery column and distilling a fluid containing dicyclopentadiene, obtained by dimerization of at least part of the cyclopentadiene, the cyclopentadiene and the C₆ to C₈ fraction. The process is **characterized in that** an overhead liquid containing cyclopentadiene and a bottom liquid containing dicyclopentadiene are obtained from the top and the bottom of the recovery column, respectively and in that the overhead liquid is refluxed so that the overhead liquid has a cyclopentadiene content of 80 to 99% by mass.
The process for producing trimethylenenorbornane according to the present invention comprises the above-described process for the production of dicyclopentadiene as its step (C) described below and preferably includes the following steps (A) to (F) in this order. More specifically, one embodiment of the dicyclopentadiene production process comprises the steps of:
(A) feeding the above-described thermally cracked gasoline to a first distillation column and carrying out distillation thereof at a column bottom temperature of 160°C or less to distil an overhead liquid containing a C₅ or lower fraction from a top of the first distillation column;
(B) feeding a bottom liquid in the first distillation column, which contains dicyclopentadiene, to a second distillation column and carrying out distillation thereof at a column bottom temperature of 165°C or higher to withdraw a bottom liquid containing a C₉ or higher fraction from a bottom of the second distillation column;
(C) introducing an overhead liquid, obtained from a top of the second distillation column in the step (B) and containing cyclopentadiene and a C₆ to C₈ fraction, into a cyclopentadiene recovery column to recover cyclopentadiene from a top thereof and dicyclopentadiene from a bottom thereof;
(D) hydrogenating the column bottom liquid obtained from the cyclopentadiene recovery column in the step (C);
(E) feeding the hydrogenated oil obtained in the step (D) to an extraction column to extract aromatic hydrocarbon compounds and to obtain an extraction residue (raffinate); and optionally
(F) feeding the extraction reside obtained in the step (E) to a raffinate fractionating column and carrying out distillation thereof to obtain a heavy raffinate heavy from a bottom thereof.
Each of the above steps will be described below.

### [Starting raw material: thermally cracked gasoline]

A starting raw material used in the dicyclopentadiene production process according to the present invention is a thermally cracked gasoline obtained by thermally cracking a petroleum hydrocarbon oil.
As the petroleum hydrocarbon oil used as the raw material of the thermally cracked gasoline, there may be mentioned, for example, naphtha, light oil, natural gas liquid, crude oil and heavy oil. When such a petroleum hydrocarbon oil is subjected to a pyrolysis treatment at a high temperature of 700°C or more, olefins such as ethylene and propylene are produced. In this case, so called thermally cracked gasoline having boiling points in the range of about 35 to 200°C is secondarily produced. In addition to olefins and diolefins, the thermally cracked gasoline generally contains 50 to 80% by mass of aromatic hydrocarbon compounds such as benzene, toluene and xylene.
The pyrolysis treatment of such a petroleum hydrocarbon oil may be carried out by any of a method using a tubular cracking furnace, a cracking method using a heating medium, a catalytic cracking method, etc.

### [Step (A)]

This step includes feeding the above-described thermally cracked gasoline to a first distillation column and carrying out distillation thereof at a column bottom temperature of 160°C or less to distill an overhead liquid containing a C₅ or lower fraction from a top of the first distillation column. In the first distillation column, distillation of the thermally cracked gasoline is carried out while controlling the column bottom temperature at a temperature not exceeding the decomposition temperature of dicyclopentadiene, namely not exceeding 160°C, to distill a C₅ or lower fraction, such as 1,3-butadiene, isopentane, n-pentane, isoprene, pentene and pentadiene, from the top of the first distillation column, thereby removing most of the C₄ and C₅ fractions. At the same time, a bottom liquid containing dicyclopentadiene is withdrawn from a bottom of the first distillation column. The column bottom temperature is preferably 140 to 160°C and the column top pressure is generally 0.35 to 0.40 MPa.

### [Step (B)]

This step includes feeding the dicyclopentadiene-containing bottom liquid in the first distillation column in the step (A) to a second distillation column and carrying out distillation thereof at a column bottom temperature of 165°C or higher to withdraw a bottom liquid containing a C₉ or higher fraction from a bottom of the second distillation column. In the second distillation column, distillation is carried out under a column top pressure of generally ambient pressure, if desired a slightly reduced or increased pressure, while controlling the column bottom temperature at a temperature at which dicyclopentadiene is preferentially decomposed, namely at least 165°C, preferably 170 to 220°C to withdraw a bottom liquid containing a C₉ or higher fraction from the bottom thereof and to distill a liquid containing cyclopentadiene and a C₆ to C₈ fraction from the top thereof. As a result, most of the C₉ fraction is removed from the bottom, while the cyclopentadiene-containing fraction is obtained from the top.
The overhead liquid obtained from the top is a liquid containing cyclopentadiene and a C₆ to C₈ fraction. Depending upon the conditions under which the step (B) is carried out, the overhead liquid may contain additional components such as C₅, C₉ and C₁₀ fractions. The overhead liquid contains cyclopentadiene in an amount of generally 2 to 10% by mass, a C₆ to C₈ fraction in an amount of generally 87.5 to 98.0% by mass and other components (C₅, C₉ and C₁₀ fractions) in an amount of generally 0 to 2.5% by mass.

### [Step (C)]

This step includes introducing the overhead liquid, obtained from the top of the second distillation column in the step (B) and containing cyclopentadiene and a C₆ to C₈ fraction, into a cyclopentadiene recovery column to recover cyclopentane from a top thereof and dicyclopentadiene from a bottom thereof. The cyclopentadiene recovery column is adapted to distill the fluid containing dicyclopentadiene, formed in situ by dimerization of at least part of cyclopentadiene, the cyclopentadiene and the C₆ to C₈ fraction while refluxing an overhead liquid so that the overhead liquid has a cyclopentadiene content of 80 to 99% by mass. Thus, dicyclopentadiene which serves as a raw material of trimethylenenorbornane can be obtained with a high efficiency without additionally using a dicyclopentadiene hydrogenation unit or a cyclopentadiene dimerization column.
Cyclopentadiene is recovered from the column top and is generally utilized as a raw material of a hydrogenated petroleum resin. The column bottom liquid obtained from the bottom of the recovery column and containing dicyclopentadiene is fed to the succeeding step (D) where it is subjected to a hydrogenation treatment to produce trimethylenenorbornane which serves as a raw material of adamantane.
Because at least part of the cyclopentadiene is dimerized into dicyclopentadiene by keeping the cyclopentadiene content of the overhead liquid within the above-described range, dicyclopentadiene can be obtained from the bottom with a high efficiency. It follows that trimethylenenorbornane can be produced with a high efficiency.

In order to obtain dicyclopentadiene in a large amount, the overhead liquid in the cyclopentadiene recovery column should be refluxed. In this case, the reflux ratio may be adjusted so that the overhead liquid has a cyclopentadiene content of 80 to 99% by mass, preferably 85 to 95% by mass.
The reflux ratio (R/D) which is a ratio of the quantity of liquid refluxed to the cyclopentadiene recovery column to the amount of the overhead liquid product withdrawn may be suitably adjusted by the plate number of the recovery column. When the plate number is 32, for example, the reflux ratio may be in the range of 7 to 16. The cyclopentadiene recovery column is preferably operated at a pressure of -0.07 to (7.2 MPa, more preferably 0 to 0.05 MPa, from the viewpoint of production of dicyclopentadiene. When the operation pressure is in the above range, the dimerization can be accelerated so that the reaction rate is improved. In order to obtain the desired cyclopentadiene content in the overhead liquid from the cyclopentadiene recovery column, the recovery column is operated by controlling single one of the reflux ratio (R/D) and the operation pressure or by controlling both of them in combination.
The column bottom temperature of the cyclopentadiene recovery column is preferably 50 to 120°C, more preferably 80 to 120°C, still more preferably 90 to 110°C. When the column bottom temperature is in the above range, the reaction rate of dimerization of cyclopentadiene is not reduced and, further, dicyclopentadiene is not decomposed.
When cyclopentadiene is contained in the column bottom liquid, cyclopentane is produced by the succeeding hydrogenation treatment. Since the cyclopentane in no way contributes to an increase of the production amount of adamantane., it is preferred that the cyclopentadiene content of the column bottom liquid be as small as possible. By controlling the operation conditions of the cyclopentadiene recovery column within the above-described ranges, the equilibrium between cyclopentadiene and dicyclopentadiene favors the latter. Therefore, the amount of cyclopentadiene in the column bottom liquid can be reduced so that the yield of cyclopentadiene recovered as the overhead liquid is not reduced.

### [Step (D)]

This step includes hydrogenating the column bottom liquid obtained from the bottom of the cyclopentadiene recovery column in the step (C) and containing dicyclopentadiene.
The column bottom liquid contains olefins in addition to dicyclopentadiene. The hydrogenation treatment can selectively hydrogenate dicylopentadiene and diolefins such as olefins into saturated hydrocarbon compounds with the simultaneous occurrence of desulfurization and denitrification thereof. Therefore, it is important that the hydrogenation conditions should be selected so that aromatic hydrocarbon compounds are prevented from being hydrogenated. In the present invention, the hydrogenation may be carried out in a single stage procedure or in a multi-stage (such as two-stage) procedure. Any hydrogenation catalyst may be used without limitation. Thus, conventionally known catalysts such as nickel-based, cobalt-based, molybdenum-based and palladium-based catalysts may be used singly or in combination of two or more thereof. In the case of a two-stage hydrogenation the first stage may be suitably carried out at a low temperature using a palladium-basted or nickel-based catalyst for the prevention of polymerization of diolefins, while the second stage may be suitably carried out using a cobait-molybdenum-based or nickel-molybdenum-based catalyst without being limited by temperature.

With regard to hydrogenation reaction conditions, the reaction temperature is generally 50 to 350°C, preferably 100 to 350°C, the reaction pressure is generally 4.0 to 6.5 MPa, preferably 4.5 to 6.0 MPa, the liquid hourly space velocity (LHSV) is generally 1 to 30 h⁻¹, preferably 1 to 10 h⁻¹, and the hydrogen gas flow rate is generally 50 to 500 Nm³/(KL-oil, preferably 200 to 400 Nm³/KL-oil.
As a result of the hydrogenation treatment, the column bottom liquid from the cyclopentadiene recovery column containing dicylopentadiene and olefins is converted into a mixture of saturated hydrocarbon compounds and aromatic hydrocarbon compounds. The column bottom liquid is occasionally referred to as platifinate.

### [Step (E)]

This step includes feeding the hydrogenated oil obtained in the step (D) to an extraction column to extract the aromatic hydrocarbon compounds and to obtain an extraction residue (raffinate).
As an extraction solvent used for the extraction in this step is not specifically limited as long as it is a polar organic solvent which can well dissolve aromatic hydrocarbon compounds but poorly dissolve non-aromatic hydrocarbon compounds. Thus, various extraction solvents may be used. It is also possible to control the extraction efficiency, etc. by incorporating water in the solvent. As the solvent extraction method, there may be mentioned, for example, a sulfolane process using sulfolane as the extraction solvent, a Udex process using an ethylene glycol, an Arosolvan process using N-methylpyrrolidone, a DMSO process using dimethylsulfoxide and a Formex process using formylmorpholine. Although any of these processes may be utilized, the sulfolane process using sulfolane is preferred.

The sulfolane process is generally carried out in the manner as described below. The hydrogenated oil obtained in the above step (D) is first introduced into an intermediate portion of a liquid-liquid extraction column and is brought into a counter-current liquid-liquid contact with a sulfolane solvent supplied from a column top portion so that aromatic hydrocarbon compounds contained in the hydrogenated oil are mainly extracted with the sulfolane. The extracted liquid stream (so-called rich solvent) containing the solvent and the main extract, namely aromatic hydrocarbon compounds, is withdrawn from the bottom of the extraction column, while the extraction residue termed raffinate is withdrawn overhead from the column.
As the liquid-liquid extraction column, a counter-current multi-stage extraction column is preferred. For example, a counter-current rotary disc extraction column may be used. The extraction conditions may include a sulfolane to hydrogenated oil volume ratio of 0.5 to 10, for example 2.6, and an extraction temperature of 50 to 150°C, for example 70°C. The operation pressure (at column bottom) is not specifically limited as long as the pressure is sufficient for maintaining a liquid phase for the liquid-liquid extraction, but may be generally 0.3 to 1 MPa, for example 0.5 MPa. In the manner described above, a raffinate which is almost free of aromatic hydrocarbon compounds and which contains, as its main component, saturated hydrocarbon compounds including trimethylenenorbornane is obtained.

### [Step (F)]

This step includes feeding the extraction reside (raffinate) obtained in the step (E) to a raffinate fractionating column, carrying out distillation thereof to obtain a heavy raffinate from a bottom thereof, and feeding the heavy raffinate to a heavy raffinate fractionating column to obtain a heavy raffinate heavy.
The raffinate fractionating column is provided for distilling the raffinate for separating most of C₅ or lower fraction as distillate and withdrawing a heavy raffinate mainly composed of C₆ or higher fraction from the column bottom. The column bottom temperature of the raffinate fractionating column is generally 90 to 100°C. The column bottom liquid (heavy raffinate) of the raffinate fractionating column is fed to the heavy raffinate fractionating column and is separated into a C₇ or lower fraction as an overhead liquid, a C₇ to C₉ fraction as an intermediate distillate and a heavy raffinate heavy (containing trimethylenenorbornane, methyltrimethylenenorbornane and, additionally, C₉ to C₁₁ naphthenes and aromatics) withdrawn from the column bottom. The column bottom temperature is generally 190 to 210°C. The column top pressures in the raffinate fractionating column and the heavy raffinate fractionating column are generally ambient pressure but, if necessary, may each be a slightly reduced or slightly increased pressure.
As a raw material of adamantane, the raffinate obtained in the step (E) and the heavy raffinate heavy obtained in the step (F) may be used singly or in combination. As the process for producing adamantane using such a raw materials there may be mentioned a production process using isomerization of trimethylenenorbornane contained in a raffinate (for example, pamphlet of International Publication No. WO05/058779)

### [Examples]

The present invention will be next described in more detail with reference to examples but is not deemed to be limited thereto in any way.

### Example 1

Naphtha was subjected to a pyrolysis treatment at 790 to 840°C in a tubular cracking furnace to obtain a thermally cracked gasoline having a boiling point of 35 to 200°C. The thermally cracked gasoline was composed of 9.3% by mass of paraffins, 33.9% by mass of unsaturated hydrocarbon compounds, 2.0% by mass of naphthenes and 54.8% by mass of aromatic hydrocarbon compounds. The thermally cracked gasoline was then distilled in a de-pentane column (first distillation column, column bottom temperature: 159°C) and a rerun column (second distillation column, column bottom temperature: 190°C) to obtain a bottom liquid containing C₉ or higher fraction and an overhead liquid containing cyclopentadiene and C₆ to C₈ fraction. The thus obtained overhead liquid containing cyclopentadiene and C₆ to C₈ fraction was fed to a cyclopentadiene recovery column having a theoretical plate number of 24 and refluxed at a reflux ratio (R/D, a ratio of the quantity of liquid refluxed to the amount of the overhead liquid product withdrawn) of 7.8 to obtain a column bottom liquid containing 2.0% by mass of cyclopentadiene and 0.32% by mass of dicyclopentadiene and an overhead liquid containing 93.0% by mass of cyclopentadiene and 1.3% by mass of dicyclopentadiene. The cyclopentadiene recovery column was operated at a pressure of 0.008 MPa and a column bottom temperature of 91°C. The column bottom liquid from the cyclopentadiene recovery column was subjected to a two-stage hydrogenation treatment for the selective hydrogenation of olefins and dienes. The first stage was carried out using a palladium-based catalyst at a reaction temperature of 110°C, an LHSV of 3.0 h⁻¹ and a hydrogen gas flow rate of 280 Nm³/KL-oil, while the second stage was carried out using a cobalt-molybdenum-based catalyst at a reaction temperature of 300°C, an LHSV of 2.5 h⁻¹ and a hydrogen gas flow rate of 330 Nm³/KL-oil.

The thus obtained hydrogenated, thermally cracked gasoline was treated in a stripper to distill off a gas fraction such as methane and hydrogen sulfide and then subjected to solvent extraction by the sulfolane process. Thus, the hydrogenated, thermally cracked gasoline was fed to an intermediate portion of an extraction column, while supplying sulfolane to a column top thereof. Thus, in the extraction column, the thermally cracked gasoline was brought into counter-current contact with the sulfolane to selectively extract aromatic hydrocarbon compounds with the sulfolane, thereby obtaining a so-called raffinate from the top of the column. The liquid-liquid extraction was carried out at a volume ratio of the sulfolane to the hydrogenated material to be selectively treated of 2.6 and an extraction temperature of 70°C. The raffinate was next distilled at a reflux ratio of 0.7 in a distillation column (raffinate fractionating column) having a theoretical plate number of 16 to remove a light fraction, thereby obtaining a heavy raffinate heavy having properties shown in Table 1.
The cyclopentadiene content and dicyclopentadiene content of the overhead liquid obtained from the column top of the cyclopentadiene recovery column, the cyclopentadiene content and dicyclopentadiene content of the column bottom liquid, the trimethylenenorbornane content in the raffinate obtained in Example 1, and the column, bottom temperature, content, of the cyclopentadiene are shown in Table 1. The cyclopentadiene content of the overhead liquid (containing cyclopentadiene and a C₆ to C₈ fraction) supplied to the cyclopentadiene recovery column is also shown In Table 1.

### Example 2

A heavy raffinate heavy was obtained in the same manner as in Example 1 except that the reflux ratio of the cyclopentadiene recovery column was changed to 15.6. The cyclopentadiene content and dicyclopentadiene content of the overhead liquid obtained from the column top of the cyclopentadiene recovery column, the cyclopentadiene content and dicyclopentadiene content of the column bottom liquid, the trimethylenenorbornane content in the raffinate obtained in Example 2, and the column bottom temperature, content, of the cyclopentadiene are shown in Table 1. The cyclopentadiene content of the overhead liquid (containing cyclopentadiene and a C₆ to C₈ fraction) supplied to the cyclopentadiene recovery column is also shown In Table 1.

### Example 3

A heavy raffinate heavy was obtained in the same manner as in Example 1 except that the reflux ratio of the cyclopentadiene recovery column was changed to 10.3. The cyclopentadiene content and dicyclopentadiene content of the overhead liquid obtained from the column top of the cyclopentadiene recovery column, the cyclopentadiene content and dicyclopentadiene content of the column bottom liquid, the trimethylenenorbornane content in the raffinate obtained in Example 3, and the column bottom temperature, content, of the cyclopentadiene are shown in Table 1. The cyclopentadiene content of the overhead liquid (containing cyclopentadiene and a C₆ to C₈ fraction) supplied to the cyclopentadiene recovery column is also shown In Table 1.

### Comparative Example

A heavy raffinate heavy was obtained in the same manner as in Example 1 except that the cyclopentadiene recovery column was bypassed. The cyclopentadiene content and dicyclopentadiene content of the overhead liquid obtained from the column top of the cyclopentadiene, recovery column, the cyclopentadiene content and dicyclopentadiene content of the column bottom liquid, and the trimethylenenorbornane content in the raffinate obtained in Comparative Example are shown in Table 1. The cyclopentadiene content of the overhead liquid (containing cyclopentadiene and a C₆ to C₈ fraction) supplied to the cyclopentadiene recovery column is also shown In Table 1.

**[Table 1]**

| | | Example 1 | Example 2 | Example 3 | Comparative Example |
|---|---|---|---|---|---|
| Operation conditions of cyclopentadiene, recovery column | | | | | bypass |
| | Reflux ratio (R/D) | 7.8 | 15.6 | 10.3 | - |
| | Column bottom temperature (°C) | 91 | 95 | 104 | - |
| | Operation pressure (MPa) | 0.02 | 0.02 | 0.07 | - |

| Raffinate | | | | | |
|---|---|---|---|---|---|
| | Trimethylenenorbornane content (% by mass) | 0.9 | 1.3 | 1.8 | 0.3 |

| Liquid feed to cyclopentadiene recovery column | | | | | |
|---|---|---|---|---|---|
| | Cyclopentadiene content (% by mass) | 5.0 | 5.0 | 5.0 | 5.0 |

| Overhead liquid from cyclopentadiene recovery column | | | | | |
|---|---|---|---|---|---|
| | Cyclopentadiene content (% by mass) | 93.0 | 94.0 | 91.9 | 5.0 |
| | Dicyclopentadiene content (% by mass) | 1.3 | 1.7 | 0.55 | 0.32 |

| Bottom liquid from cyclopentadiene recovery column | | | | | |
|---|---|---|---|---|---|
| | Cyclopentadiene content (% by mass) | 2 | 1 | 1 | 5 |
| | Dicyclopentadiene content (% by mass) | 0.89 | 1.3 | 1.8 | 0.32 |

### [Industrial Applicability]

According to the process for producing trimethylenenorbornane of the present invention, it is possible to produce trimethylenenorbornane, which serves as a raw material of adamantane, with a high efficiency in a raffinate or a heavy raffinate heavy by producing dicyclopentadiene, which serves as a raw material of trimethylenenorbornane, with a high efficiency without additionally using a dicyclopentadiene hydrogenation unit or a cyclopentadiene dimerization column.

## Claims

1. A process for the production of dicyclopentadiene, comprising introducing a liquid feed containing cyclopentadiene and a C₆ to C₈ fraction into a cyclopentadiene recovery column, and carrying out distillation of a fluid containing dicyclopentadiene, obtained by dimerization of at least part of the cyclopentadiene, the cyclopentadiene and the C₆ to C₈ fraction, wherein an overhead liquid containing cyclopentadiene and a bottom liquid containing dicyclopentadiene are obtained from the top and the bottom of the recovery column, respectively, and the overhead liquid is refluxed so that a cyclopentadiene content of the overhead liquid is 80 to 99% by mass.

2. The process for the production of dicyclopentadiene as recited in claim 1, wherein a column bottom temperature of the recovery column is 50 to 120°C.

3. The process for the production of dicyclopentadiene as recited in claim 1 or 2, wherein the liquid feed containing cyclopentadiene and a C₆ to C₈ fraction has a cyclopentadiene content of 2 to 10% by mass.

4. A process for producing trimethylenenorbornane comprising the process for the production of dicyclopentadiene as recited in any one of claims 1 to 3.
